# EUROPEAN PATENT SPECIFICATION

(11) **EP 3 875 575 B1**
(45) Date of publication and mention of the grant of the patent: **17.04.2024**
(21) Application number: 19879796.1
(22) Date of filing: 16.10.2019
(51) Int. Cl.: C12M 3/00, C12M 3/06, B01L 3/00

(54) **MICRO-FLUID CHIP AND MICRO-FLUID DEVICE**
MIKROFLUIDCHIP UND MIKROFLUIDVORRICHTUNG
PUCE MICROFLUIDIQUE ET DISPOSITIF MICROFLUIDIQUE

(30) Priority: 29.10.2018 JP 2018202991
(43) Date of publication of application: 08.09.2021
(73) Proprietor: NOK Corporation, Minato-ku, Tokyo 105-8585 (JP); The University of Tokyo, Bunkyo-ku, Tokyo 113-8654 (JP)
(72) Inventor: YOSHITOMI Takumi, Fujisawa-shi, Kanagawa 251-0042 (JP); KIM SooHyeon, Tokyo 113-8654 (JP); FUJII Teruo, Tokyo 113-8654 (JP); SUN Mingyue, Tokyo 113-8654 (JP)
(74) Representative: Global IP Europe Patentanwaltskanzlei
(86) International application number: PCT/JP2019/040737
(87) International publication number: WO 2020/090481

(56) References cited:
- EP-A1- 3 078 421
- WO-A1-2012/060186
- WO-A1-2019/069900
- CN-A- 108 704 681
- JP-A- 2005 257 283
- US-A1- 2006 159 601
- ROGAL, J. et al.: "Integration concepts for multi-organ chips: how to maintain flexibility?!", Future science OA, vol. 3, no. 2, 13 March 2017 (2017-03-13), page FS0180, XP055527584,
- SON, MEIGETSU et al.: "Modularization of biofunctional chips using microfluidic devices manufactured by 3D printer", Lecture abstracts of 38th CHEMINAS; October 30 - November 1, 2018, 30 October 2018 (2018-10-30), page 79, XP009526032,

## Description

### TECHNICAL FIELD

The present invention relates to microfluidic chips and to microfluidic devices.

### BACKGROUND ART

Document JP2005 257 283 A discloses a microfluidic chip according to the preamble of claim1. Microfluidic devices are devices that have minute flow passages through which liquids flow for analysis or mixing. As one type of microfluidic chip that constitutes a microfluidic device, there has been developed an organ-on-a-chip that simulates an organ or tissue of an animal, and in which cells can be cultured in a channel (Non-Patent Document 1).

An organ-on-a-chip is used, for example, as an alternative to an animal experiment in the field of drug development. Animal experiments are useful in assessing effects and side effects of drugs, but are undesirable from a viewpoint of animal welfare. Furthermore, animal experiments suffer from a drawback in that while they may be effective for evaluation of an effect of a drug on one type of animal, such evaluation may not be applicable to other types of animal. For example, results of experiments in which a drug is administered to mice may not provide a full understanding of the effects and side effects the same drug in humans. To investigate an effect of a drug on an animal, it is desirable to use an organ-on-a-chip for culture of cells derived from the animal.

### BACKGROUND DOCUMENTS

### Non-patent Documents

Non-patent Document 1: Julia Rogal, et al., "Integration concepts for multi-organ chips: how to maintain flexibility?!", Future Science OA, 2017, [online], [Searched on September 10, 2018], Internet (URL: https://www.ncbi.nlm.nih.gov/pmc/articles/PMC5481865/)

### SUMMARY OF THE INVENTION

The present invention provides a microfluidic chip according to claim 1 and microfluidic devices comprising said microfluidic chip.

The microfluidic chip is inserted into the slot along a horizontal direction, so that the microfluidic device can be easily formed of a member that includes the slot and the microfluidic chip. For example, in order to evaluate human drug metabolism by use of an organ-on-a-chip, it is preferable to culture a plurality of different cell types. In a case in which a plurality of types of cells are cultured on a single chip, the function of the entire device will be lost if cultivation of only one type of cell fails. According to one example of the present invention, cells of different types can be respectively cultivated on different microfluidic chips, and only microfluidic chips in which cultivation has been successfully completed can be selected. Further, the microfluidic chips can be mounted to a member that includes a plurality of slots, and thus the microfluidic chips can be easily connected to each other.

### BRIEF DESCRIPTION OF THE DRAWINGS

Fig. 1 is a plan view of a microfluidic device according to an embodiment of the present invention;
Fig. 2 is a plan view showing elements disassembled from the microfluidic device shown in Fig. 1;
Fig. 3 is a plan view showing a connection flow passage chip in the microfluidic device shown in Fig. 1;
Fig. 4 is a cross-sectional view of the connection flow passage chip taken along line IV-IV in Fig. 3;
Fig. 5 is a plan view showing a microfluidic chip of the microfluidic device shown in Fig. 1;
Fig. 6 is a cross-sectional view of the microfluidic device taken along line VI-VI in Fig. 1;
Fig. 7 is a back view showing a flow passage plate of the microfluidic chip shown in Fig. 5;
Fig. 8 is a side cross-sectional view showing a microfluidic device different from the embodiment;
Fig. 9 is an enlarged cross-sectional view of the microfluidic chip taken along line IX-IX in Fig. 7;
Fig. 10 is an enlarged back view showing a part of the flow passage plate of the microfluidic chip;
Fig. 11 is perspective view showing an arrangement of elements in the microfluidic chip;
Fig. 12 is a side cross-sectional view showing a microfluidic device according to a modification of the embodiment;
Fig. 13 is a side cross-sectional view showing a microfluidic device according to another modification of the embodiment;
Fig. 14 is an enlarged back view showing a part of the flow passage plate of a microfluidic chip according to another modification of the embodiment;
Fig. 15 is an enlarged back view showing a part of the flow passage plate of a microfluidic chip according to another modification of the embodiment;
Fig. 16 is a side cross-sectional view showing a microfluidic device according to another modification of the embodiment;
Fig. 17 is a side cross-sectional view showing a microfluidic device according to another modification of the embodiment;
Fig. 18 is a side cross-sectional view showing a phase immediately before the microfluidic chip according to the embodiment is inserted into a slot of a holder;
Fig. 19 is a side cross-sectional view showing a first phase at which the microfluidic chip according to the embodiment is inserted into the slot of the holder;
Fig. 20 is a side cross-sectional view showing the next phase of Fig. 19;
Fig. 21 is a side cross-sectional view showing the next phase of Fig. 20;
Fig. 22 is a side cross-sectional view showing the next phase of Fig. 21;
Fig. 23 is a side cross-sectional view showing the next phase of Fig. 22;
Fig. 24 is a side cross-sectional view showing a first phase at which a microfluidic chip according to a comparative example is inserted into a slot of a holder;
Fig. 25 is a side cross-sectional view showing a phase after Fig. 24;
Fig. 26 is a side cross-sectional view showing a microfluidic device according to another modification of the embodiment;
Fig. 27 is a side cross-sectional view showing a microfluidic device according to another modification of the embodiment; and
Fig. 28 is a plan view showing a microfluidic device according to another modification of the embodiment.

### DESCRIPTION OF EMBODIMENT

Hereinafter, with reference to the accompanying drawings, an embodiment according to the present invention will be described. It is of note that the drawings may not necessarily accurately show relative dimensional ratios of actual products according to the embodiment(s) and certain dimensions may be exaggerated.

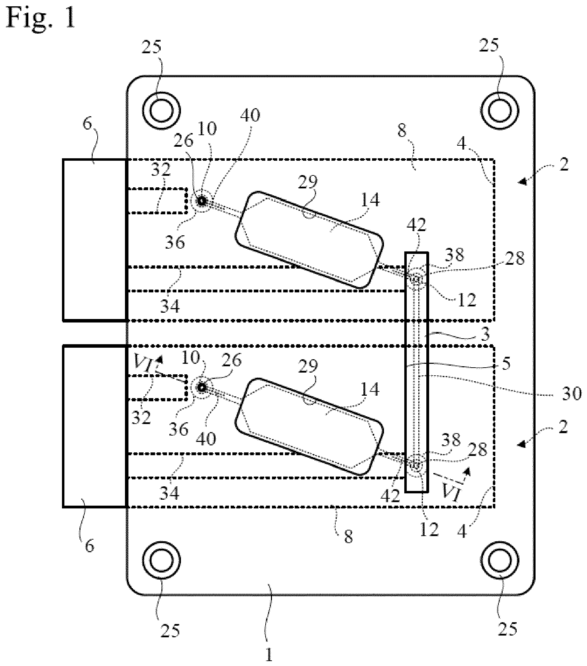

A microfluidic device according to the embodiment is an organ-on-a-chip device. As shown in Fig. 1, the microfluidic device includes a holder 1, a plurality of (two in this embodiment) microfluidic chips 2 held by the holder 1, and a connection flow passage chip 3 that is held by the holder 1 and connects the microfluidic chips 2.

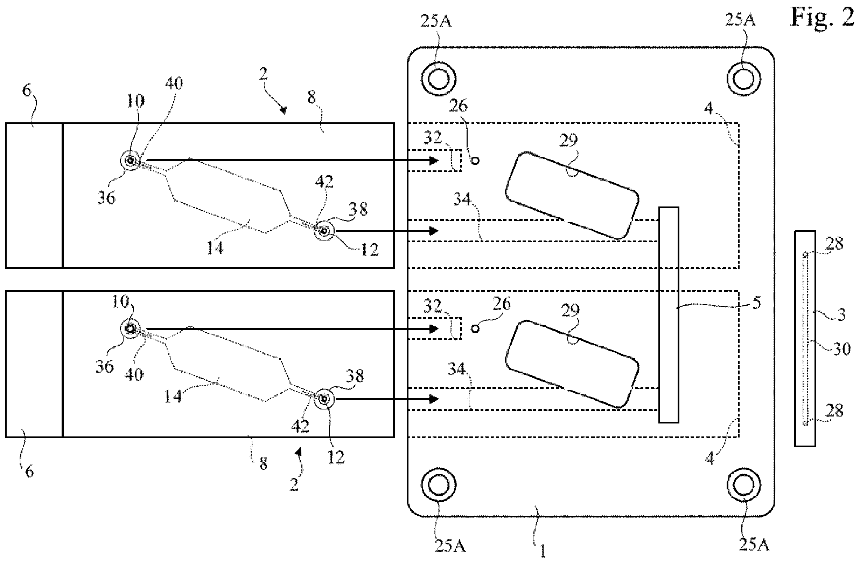

Each of the microfluidic chips 2 is constituted of a rectangular plate. As shown in Figs. 1 and 2, the holder 1 is constituted of a substantially rectangular plate having a plurality of horizontally extending slots 4 into which the microfluidic chips 2 are respectively inserted along a horizontal direction. In addition, a rectangular elongated opening 5, into which the rectangular elongated connection flow passage chip 3 is fitted, is formed at the upper portion of the holder 1.

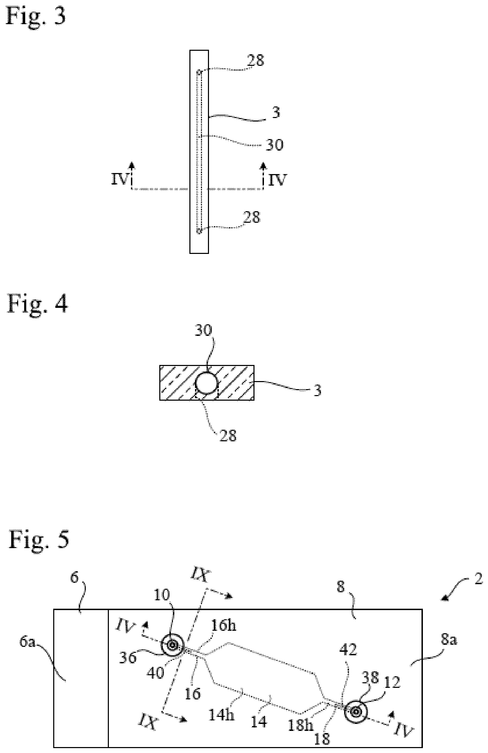

As shown in Figs. 3 and 4, two holes 28 are formed at respective ends of the connection flow passage chip 3, and a connection flow passage 30 that connects each of the holes 28 is formed at the center of the connection flow passage chip 3. The connection flow passage 30 extends along the longitudinal direction of the connection flow passage chip 3. The two holes 28 do not penetrate the connection flow passage chip 3, and open at a surface of the connection flow passage chip 3.

The connection flow passage chip 3 is formed from a transparent elastomer such as silicone rubber or acrylic rubber of which the main component is polydimethylsiloxane (PDMS). The method of manufacturing the connection flow passage chip 3 is not limited, but the connection flow passage chip 3 can be manufactured by use of, for example, a 3D printer. Specifically, the outer shape of the connection flow passage chip 3 is formed from an ultraviolet curable PDMS around a support material (core) that forms the holes 28 and the connection flow passage 30 by use of, for example, a stereolithography method. Thereafter, the holes 28 and the connection flow passages 30 can be opened by dissolving the support material with an alkaline solution and removing the support material. However, the connection flow passage chip 3 may be manufactured by joining parts molded in molds that are formed by use of photolithography or a 3D printer.

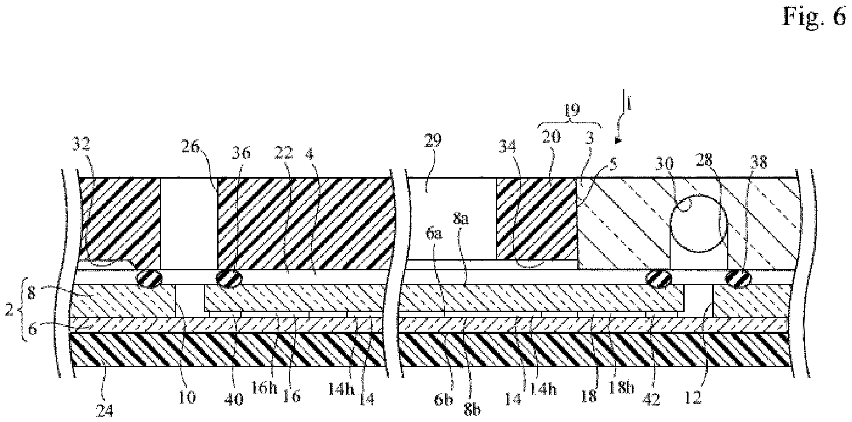

As shown in Figs. 5 and 6, each of the microfluidic chips 2 has a rectangular flat plate 6 of uniform thickness, and a flow passage plate 8 that is a rectangular flat plate of uniform thickness stacked on the flat plate 6. The flat plate 6 has a flat upper surface 6a and a flat lower surface 6b parallel to the upper surface 6a. The flow passage plate 8 has a flat upper surface 8a and a flat lower surface 8b parallel to the upper surface 8a. The flat plate 6 and the flow passage plate 8 are arranged horizontally, and the flow passage plate 8 is arranged above the flat plate 6. The upper surface 6a of the flat plate 6 is joined to the lower surface 8b of the flow passage plate 8. Thus, the upper surface 8a of the flow passage plate 8 constitutes the upper surface of the microfluidic chip 2, and the lower surface 6b of the flat plate 6 constitutes the lower surface of the microfluidic chip 2.

The flat plate 6 is formed of a transparent material such as glass or acrylic resin. The flow passage plate 8 is formed from a transparent elastomer such as silicone rubber or acrylic rubber of which the main component is PDMS. The thickness of the flat plate 6 is, for example, 1 mm, whereas the thickness of the flow passage plate 8 is, for example, 2 mm, and the total thickness of the microfluidic chip 2 is, for example, 3 mm.

The flat plate 6 has a length that is greater than that of the flow passage plate 8. After the microfluidic chip 2 is inserted into the slot 4, as shown in Fig. 1, the entirety of the flow passage plate 8 and a part of the flat plate 6 are disposed inside the slots 4, while the end of the flat plate 6 remains exposed.

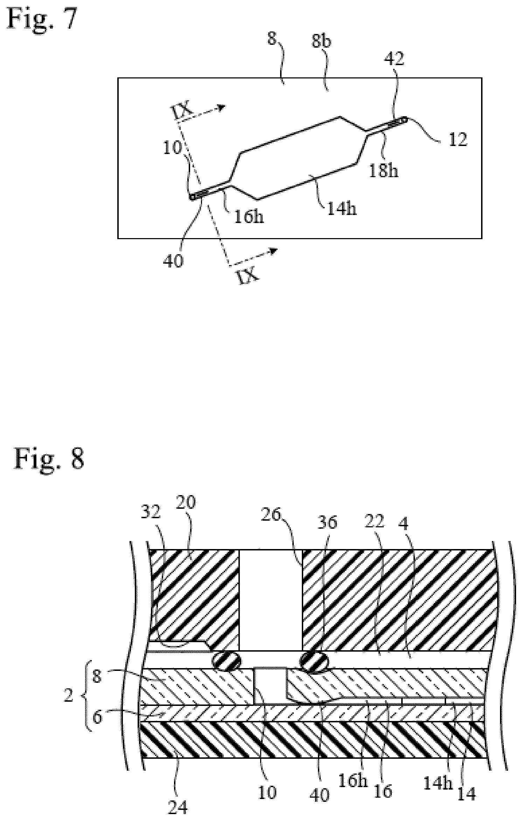

As shown in Figs. 5 and 7, a recess that serves as a liquid flow passage is formed on the lower surface 8b of the flow passage plate 8. The recess has a culture chamber recess 14h, and passage recesses 16h and 18h that communicate with respective ends of the culture chamber recess 14h. The flow passage plate 8 is formed with a communication hole 10 that communicates with the passage recess 16h, and a communication hole 12 that communicates with the passage recess 18h. The communication holes 10 and 12 open at the upper surface 8a.

The flat plate 6 is joined to the flow passage plate 8 to cover the recess and cooperates with the flow passage plate 8 to define a flow passage. In this embodiment, the flow passage includes a culture chamber 14 in which cells of animals (including humans) are cultured, a passage 16 interposed between the communication hole 10 and the culture chamber 14, and a passage 18 interposed between the communication hole 12 and the culture chamber 14. The passages 16 and 18 have a smaller width than that of the culture chamber 14. The passages 16 and 18 are of the same width and length as each other.

The culture chamber 14 is a long space that extends along a straight line, and the passages 16 and 18 extend along the same straight line. The flow passage having the culture chamber 14 and the passages 16 and 18 extends linearly (without any bend or curve) from one communication hole to the other communication hole, and thus the structure of the flow passage is simple. However, the culture chamber 14 and the passages 16 and 18 may be bent or curved. In each microfluidic chip 2, the line segment connecting the two communication holes 10 and 12 arranged at respective ends of the flow passage is inclined with respect to the insertion direction along which the microfluidic chips 2 are inserted into the slots 4. The culture chamber 14 and the passages 16 and 18 extend linearly along this line segment.

Thus, each of the microfluidic chips 2 has a flow passage for fluid arranged between the upper surface 8a and the lower surface 6b, and the two communication holes 10 and 12 that communicate with the flow passage and open at the upper surface 8a.

As shown in Fig. 6, the holder 1 has a laminated upper wall 20, middle wall 22, and lower wall 24. The ceiling surface of the slot 4 is the lower surface of the upper wall 20, whereas the bottom surface of the slot 4 is the upper surface of the lower wall 24. After the microfluidic chips 2 are inserted into the slots 4, the upper wall 20 faces the upper surfaces 8a of the flow passage plates 8 of the microfluidic chips 2, whereas the lower wall 24 faces the lower surfaces 6b of the flat plates 6 of the microfluidic chips 2. The middle wall 22 is arranged between the upper wall 20 and the lower wall 24 and defines the sides of each slot 4.

The upper wall 20, the middle wall 22, and the lower wall 24 are fixed to one another. For example, multiple fasteners 25 may be used for fixing, as shown in Fig. 1. The fasteners 25 may each include, for example, a bolt inserted into a through-hole 25A (see Fig. 2) that penetrates the upper wall 20, the middle wall 22, and the lower wall 24, and a nut that engages with the bolt. Alternatively, each of the fasteners 25 may be configured as a clamp mechanism. Alternatively, the upper wall 20, the middle wall 22, and the lower wall 24 may be integrally molded. Alternatively, the upper wall 20, the middle wall 22, and the lower wall 24 may be joined by an adhesive, a chemical reaction, or a thermal reaction.

The upper wall 20, the middle wall 22, and the lower wall 24 are made of, for example, an opaque resin material, but may be made of a transparent material.

As shown in Figs. 1, 2, and 6, the upper wall 20 has holes 26 formed therein. The holes 26 penetrate the upper wall 20 and serve as inlets or outlets for the liquid, or as outlets for air that is displaced under flow of the liquid.

An opening 5 is formed in the upper wall 20. The opening 5 penetrates the upper wall 20, and the connection flow passage chip 3 is fitted in and fixed to the opening 5. The holes 28 formed in the connection flow passage chip 3 fitted in the opening 5 open inside the slots 4. Although not shown, a fastener for fixing the connection flow passage chip 3 to the opening 5 may be used.

The connection flow passage chip 3 can also be considered to be a part of the holder 1. In other words, the holder 1 can be considered to include the upper wall structure 19 having the upper wall 20 and the connection flow passage chip 3, the middle wall 22, and the lower wall 24. The ceiling surface of the slot 4 is the lower surface of the upper wall structure 19, whereas the bottom surface of the slot 4 is the upper surface of the lower wall 24.

Moreover, a plurality of observation windows 29 are formed in and penetrate the upper wall 20. After the microfluidic chips 2 are inserted into the slots 4, the observation windows 29 coincide with the culture chamber 14 of a respective one of the microfluidic chips 2. The cells in the culture chambers 14 can be observed through the observation windows 29. For example, a microscope can be used to observe the cells. In order to increase an amount of light for observing cells, a lighting window penetrating the lower wall 24 may be formed in the lower wall 24. In this embodiment, the upper wall 20 is formed of an opaque material. However, in a case that the upper wall 20 is formed of a transparent material, it is also preferable that the observation windows 29 be formed in and penetrate the upper wall 20 so as to facilitate observation of the cells with a microscope.

A plurality of grooves 32 and 34 are formed on the lower surface of the upper wall 20. The grooves 32 and 34 extend linearly along the longitudinal direction of the slots 4 (the insertion direction along which the microfluidic chips 2 are to be inserted into the slots 4). As shown in Figs. 1 and 2, a hole 26 exists on the extension line of each groove 32, whereas a hole 28 exists on the extension line of each groove 34. When the microfluidic chips 2 are inserted into the slots 4, the communication holes 10 of the microfluidic chips 2 move within the slots 4 while overlapping the groove 32, and the communication hole 12 of the microfluidic chips 2 moves within the slots 4 while overlapping the groove 34. The groove 34 corresponding to the extension line on which the hole 28 located on the deep side of the slot 4 exists extends across the observation window 29.

After the microfluidic chips 2 are inserted into the slots 4, the multiple holes 26 formed in the upper wall 20 of the upper wall structure 19 are substantially coaxially aligned with and communicate with the communication holes 10 of the flow passage plates 8 of the microfluidic chips 2; whereas the multiple holes 28 formed in the connection flow passage chip 3 of the upper wall structure 19 are substantially coaxially aligned with and communicate with the communication holes 12 of the flow passage plates 8 of the microfluidic chips 2.

As shown in Fig. 1, the two holes 28 are connected by the connection flow passage 30. The connection flow passage 30 extends horizontally over the two slots 4. Liquid can be transferred from the flow passage of one of the microfluidic chips 2 to the flow passage of another of the microfluidic chips 2 via the connection flow passage 30.

A plurality of annular seals 36 and 38 made of an elastomer are disposed on the upper surface 8a of the flow passage plate 8, at which the communication holes 10 and 12 open. In this embodiment, the annular seals 36 and 38 are O-rings, but the seals may be rings of another cross-sectional shape, such as D-rings. The material of the annular seals 36 and 38 is, for example, silicone rubber, but other elastomers may be used. In this embodiment, the annular seals 36 and 38 are of the same size, but they may be of different sizes.

In this embodiment, as shown in Fig. 6, concave grooves into which the annular seals 36 and 38 are fitted are formed on the upper surface 8a of the flow passage plate 8. The annular seal 36 or 38 is fitted into each concave groove, and the annular seals 36 and 38 are fixed to the upper surface 8a. The annular seals 36 and 38 may be bonded to the upper surface 8a. As a bonding method, for example, adhesion with an adhesive may be used. Alternatively, two members may be chemically bonded by irradiation with oxygen plasma or by irradiation with ultraviolet rays under vacuum to activate the surfaces of the members.

The annular seal 36 is aligned substantially coaxially with the communication hole 10 formed in the flow passage plate 8 to surround the communication hole 10, whereas the annular seal 38 is aligned substantially coaxially with the communication hole 12 formed in the flow passage plate 8 to surround the communication hole 12. A part of the annular seal 36 overlaps the passage 16 whereas a part of the annular seal 38 overlaps the passage 18.

After the microfluidic chips 2 are inserted into the slots 4, the annular seal 36 is aligned substantially coaxially with and surrounds the hole 26 formed in the upper wall 20, whereas the annular seal 38 is aligned substantially coaxially with and surrounds the hole 28 formed in the connection flow passage chip 3. Therefore, the annular seal 36 surrounds the hole 26 formed in the upper wall 20 and the communication hole 10 formed in the flow passage plate 8, whereas the annular seal 38 surrounds the hole 28 formed in the connection flow passage chip 3 and the communication hole 12 formed in the plate 8.

When the microfluidic chips 2 are inserted into the slots 4, the lower surface 6b of the flat plate 6 of each of the microfluidic chips 2 slides on the lower wall 24 of the holder 1 whereas the annular seals 36 and 38 fixed to the upper surface 8a of the flow passage plate 8 slide on the upper wall 20 of the holder 1. After the microfluidic chips 2 are inserted into the slots 4, the lower wall 24 remains in surface contact with the flat plates 6 of the microfluidic chips 2. After the microfluidic chips 2 are inserted into the slots 4, the upper wall 20 of the holder 1 and the connection flow passage chip 3 face the upper surfaces 8a of the flow passage plates 8 of the microfluidic chips 2, and continuously compress the annular seals 36 and 38 toward the flow passage plate 8.

In this way, the annular seal 36 or 38 is used to surround the hole 26 or 28 formed in the upper wall 20 or the connection flow passage chip 3 and to surround the communication hole 10 or 12 formed in the flow passage plate 8. By being compressed, the annular seal 36 or 38 seals the liquid flowing from the hole 26 or 28 to the communication hole 10 or 12, or the liquid flowing from the communication hole 10 or 12 to the hole 26 or 28.

Upon compression of the annular seals 36 and 38, the flow passage plate 8 receives a reaction force from the annular seals 36 and 38. Therefore, as shown in Fig. 8, when there is nothing in the passages 16 and 18, the passage plate 8 may elastically deform and close the passages 16 and 18. Fig. 8 shows a state in which a closed portion 16A is generated in the passage 16 caused by elastic deformation of the flow passage plate 8 under a force received from the annular seal 36. Although not shown, the passage 18 may also have a closed portion caused by the elastic deformation of the flow passage plate 8 under a force received from the annular seal 38.

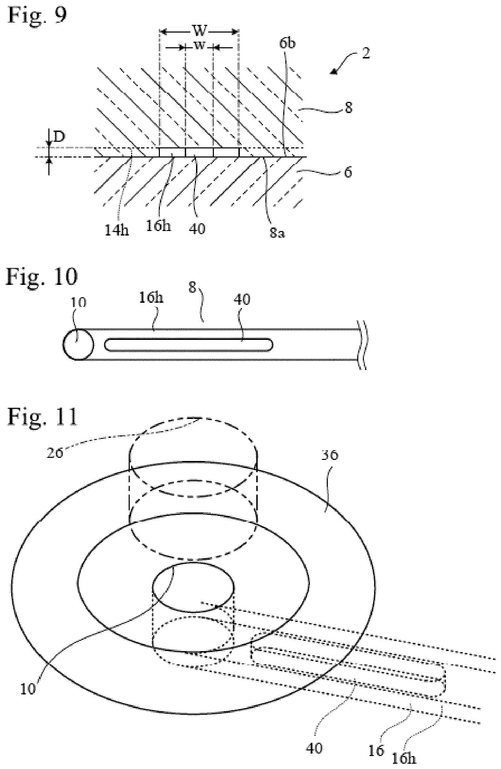

Accordingly, in this embodiment, as shown in Figs. 9 to 11, a support structure 40 that secures a height of the passage 16 so as prevent blockage of the passage 16 is fixed at a position that overlaps the annular seal 36 inside the passage 16. In addition, a support structure 42 that secures a height of the passage 18 so as to prevent blockage of the passage 18 is fixed at a position that overlaps the annular seal 38 inside the passage 18. Figs. 9 to 11 show the support structure 40 provided in the passage 16; but in Figs. 9 to 11, the passage 16, the passage recess 16h, the support structure 40, the communication hole 10, and the hole 26 may be respectively read as the passage 18, the passage recess 18h, the support structure 42, the communication hole 12 and the hole 28.

The support structures 40 and 42 are long, substantially rectangular parallelepiped projections, and are formed on the flow passage plate 8. The support structure 40 is arranged at the center in the widthwise direction of the elongated passage recess 16h forming the passage 16, and extends along the longitudinal direction of the passage recess 16h. The support structure 42 is also arranged at the center in the widthwise direction of the elongated passage recess 18h forming the passage 18, and extends along the longitudinal direction of the passage recess 18h.

The depth D of the passage recesses 16h and 18h (the depth of the culture chamber recess 14h) is, for example, 0.1 mm, and the height of the support structures 40 and 42 may be the same as the depth D.

The ratio w/W of the width w of the support structures 40 and 42 to the width W of the passage recesses 16h and 18h is not limited, but is preferably, for example, 0.2 to 0.5. If w/W is too large, the flow of liquid in the passages 16 and 18 is hindered, resulting in a significant pressure loss. For example, the width W of the passage recesses 16h and 18h may be 1 mm, and the width w of the support structures 40 and 42 may be 0.4 mm. In this case, the ratio w/W is 0.4.

Upon compression of the annular seals 36 and 38, the flow passage plate 8 receives a reaction force from the annular seals 36 and 38, but the support structures 40 and 42 fixed at positions that overlap the annular seals 36 and 38 inside the passages 16 and 18 maintain the height of the passages 16 and 18 and thereby prevent blockage of the passages 16 and 18. In this embodiment, the two annular seals 36 and 38 seal two portions, and the two support structures 40 and 42 prevent blockage of the passage at the two portions.

The flow passage plate 8 having the support structures 40 and 42 in the passage recesses 16h and 18h can be manufactured by use of, for example, soft lithography. For example, a mold for molding the flow passage plate 8 may be manufactured by forming protrusions on a substrate. Alternatively, the mold for molding the flow passage plate 8 may be manufactured by use of a 3D printer. Alternatively, the flow passage plate 8 itself may be manufactured by use of a 3D printer. In these cases, the support structures 40 and 42 in the passage recesses 16h and 18h are manufactured integrally with the flow passage plate 8 as parts of the flow passage plate 8. However, the support structures 40 and 42 may be bonded to the flow passage plate 8, in which the passage recesses 16h and 18h are formed, by use of an adhesive, a chemical reaction, or a thermal reaction.

The flow passage plate 8 may be bonded to the flat plate 6 by use of an adhesive, a chemical reaction, or a thermal reaction. For example, in a case in which the flow passage plate 8 is formed from silicone rubber containing PDMS as the main component and the flat plate 6 is formed from glass, the flow passage plate 8 may be bonded to the flat plate 6 by a siloxane bond.

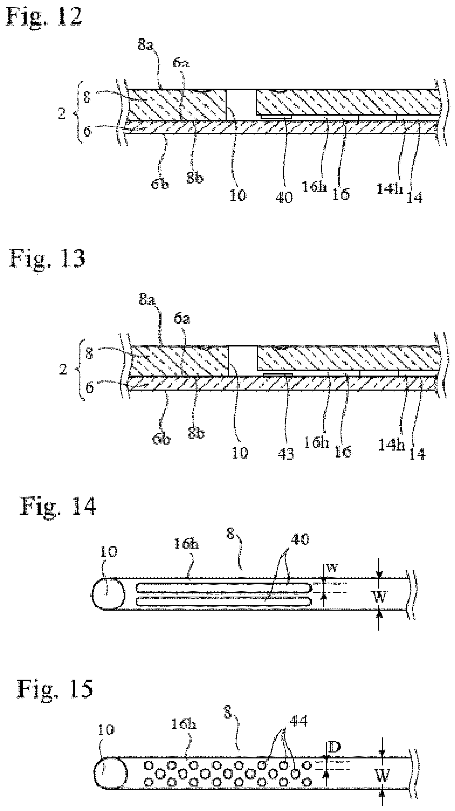

In the embodiment, the height of the support structures 40 and 42 may be the same as the depth of the passage recesses 16h and 18h. However, as shown in Fig. 12, the height of the support structure 40 may be less than the depth of the passage recess 16h, and the height of the support structure 42 may also be less than the depth of the passage recess 18h.

In the embodiment, the support structures 40 and 42 are formed on the flow passage plate 8. However, as shown in Fig. 13, a support structure 43 protruding toward the passage recess 16h may be formed on or fixed to the flat plate 6, and/or a support structure protruding toward the passage recess 18h may be formed on or fixed to the flat plate 6. Although not shown, support structures may be formed on or fixed to both the flow passage plate 8 and the flat plate 6.

In the embodiment, one support structure 40 is arranged in the passage 16, and one support structure 42 is arranged in the passage 18. However, as shown in Fig. 14, a plurality of support structures 40 may be arranged in the passage 16 and a plurality of support structures 42 may be arranged in the passage 18. In a case in which a plurality of linear support structures 40 and 42 are arranged in the passage recesses 16h and 18h, the ratio w/W of the width w of the support structures 40 and 42 to the width W of the passage recesses 16h and 18h is not limited, but is preferably, for example, 0.2/n to 0.5/n in order to achieve a smooth flow of the liquid in the passages 16 and 18, where n is the number of linear support structures arranged in one passage recess. For example, the width W of the passage recesses 16h and 18h may be 1 mm, and the width w of the support structures 40 and 42 may be 0.2 mm. In this case, n•w/W is 0.4.

Furthermore, the shapes of the support structures 40 and 42 are not limited to those of the embodiment. For example, as shown in Fig. 15, cylindrical support structures 44 may be arranged in the passages 16 and 18. In Fig. 15, the support structures 44 are arranged in three rows, in which two rows are aligned in the longitudinal direction of the passage, and one central row is offset from the other two rows. In a case in which a plurality of cylindrical support structures 44 are arranged in the passage recesses 16h and 18h, the ratio D/W of the diameter D of the support structures 44 to the width W of the passage recesses 16h and 18h is not limited, but is preferably, for example, 0.2/n to 0.5/n in order to achieve a smooth flow of the liquid in the passages 16 and 18, where n is the number of rows of the support structures 44 arranged in one passage recess and aligned in the longitudinal direction, and is 2 in the example of Fig. 15. For example, the width W of the passage recesses 16h and 18h may be 1 mm, and the diameter D of the support structures 40 and 42 may be 0.2 mm. In this case, n•D/W is 0.4.

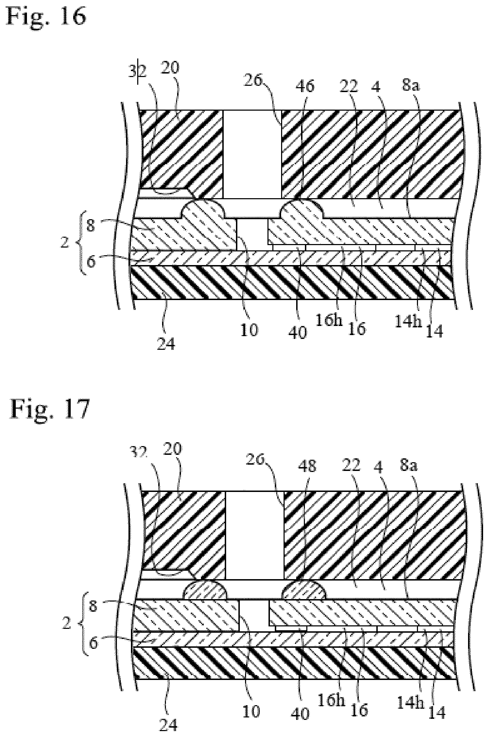

In the embodiment, the annular seals 36 and 38 are rings that are separate from the flow passage plate 8 and are fixed to the outer surface (upper surface) of the flow passage plate 8. However, as shown in Fig. 16, an annular seal 46 may be formed, as a part of the flow passage plate 8, from the same material as that of the flow passage plate 8 so as to surround the communication hole 10, and another annular seal may be formed, as a part of the flow passage plate 8, from the same material as that of the flow passage plate 8 so as to surround the communication hole 12. That is, the annular seal 46 may be a bump or lip protruding from the flow passage plate 8. In this case, the annular seal is preferably manufactured integrally with the flow passage plate 8 as part of the flow passage plate 8, for example, by use of soft lithography.

Alternatively, as shown in Fig. 17, an annular seal 48 may be formed of a material different from that of the flow passage plate 8 so as to surround the communication hole 10, and another annular seal may be formed of a material different from that of the flow passage plate 8 so as to surround the communication hole 12. In this case, the annular seals may be bonded to the outer surface (upper surface) of the flow passage plate 8 by use of an adhesive, a chemical reaction, or a thermal reaction.

Next, a usage example of the microfluidic device that is an organ-on-a-chip device according to the embodiment will be described. In the organ-on-a-chip device, one microfluidic chip 2 simulates one organ (e.g., a liver) of an animal, and another microfluidic chip 2 simulates another organ (e.g., a lung) of the animal. For example, a culture solution containing cells derived from the liver and a drug (for example, an anticancer agent) is provided in the culture chamber 14 of one microfluidic chip 2, whereas a culture solution containing lung-derived cells is provided in the culture chamber 14 of the other microfluidic chip 2. In the former culture chamber 14, the liver-derived cells produce a substance as a reaction of the drug. After a sufficient time has elapsed, the substance produced in the former culture chamber 14 is transferred to the latter culture chamber 14, and effects and side effects of the substance on the lung-derived cells can be observed through the above-described observation window 29.

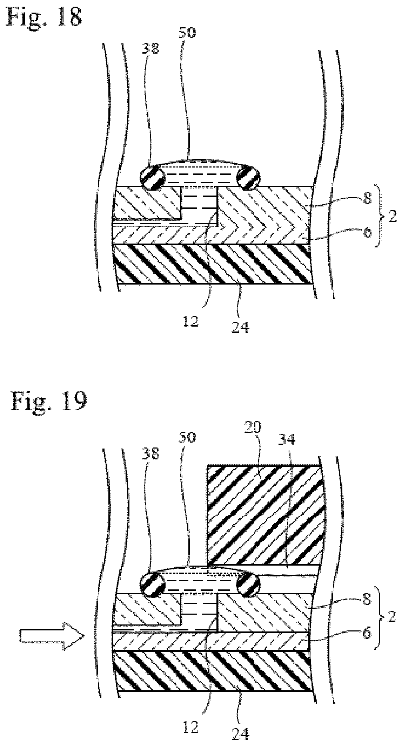

In this embodiment, after the culture solution (including cells or cells and a drug) is filled in the flow passage (the culture chamber 14 and passages 16 and 18) and the communication holes 10 and 12 of the microfluidic chip 2, it is easy to insert the microfluidic chip 2 into the slot 4 along a horizontal direction while reducing intrusion of air bubbles into the culture solution. Fig. 18 is a side cross-sectional view showing a phase immediately before the microfluidic chip 2 is inserted into the slot 4 of the holder. As shown in Fig. 18, before the microfluidic chip 2 is inserted into the slot 4, the culture solution 50 is stored not only in the flow passage and the communication holes 10 and 12, but also in the annular seals 36 and 38 that surround the communication holes 10 and 12. The upper surface of the culture solution 50 rises due to surface tension. Although Fig. 18 shows a state in which the upper surface of the culture solution 50 in the annular seal 38 surrounding the communication hole 12 is raised, the upper surface of the culture solution 50 in the annular seal 36 surrounding the communication hole 10 is also raised. In a case in which the amount of the culture solution 50 stored in the microfluidic chip 2 is small, air bubbles may intrude into the culture solution 50 when the microfluidic chip 2 is inserted into the slot 4. It is preferable that the cells in the culture solution 50 do not come into contact with air bubbles because cells of an animal are inhibited in growth or die when exposed to air. In this embodiment, the annular seals 36 and 38 are used to store a large amount of the culture solution 50, thereby reducing the intrusion of bubbles in the culture solution 50 when the microfluidic chip 2 is inserted into the slot 4.

Before the microfluidic chips 2 are inserted into the slots 4, the connection flow passage chip 3 is fixed in the opening 5 of the holder 1. Therefore, the holder 1 has, for each microfluidic chip 2, two holes 26 and 28 that communicate with the two communication holes 10 and 12, respectively. Thereafter, as shown by arrows in Fig. 2, the microfluidic chips 2 are moved horizontally toward the deep sides of the slots 4. After the microfluidic chips 2 are inserted into the slots 4, each communication hole 10 or 12 communicates with one target hole 26 or 28 in the holder 1. When the microfluidic chips 2 are inserted into the slot 4, if the communication holes 12, which are to be arranged in the deep sides of the slots 4, pass through the non-target hole 26 during the travel of the communication holes 12, bubbles may intrude into the culture solution 50. If each line segment connecting the communication holes 10 and 12, and thus, each line segment connecting the holes 26 and 28 extend along the insertion direction of the microfluidic chips 2, this situation occurs.

However, in this embodiment, each line segment that connects the two communication holes 10 and 12 is inclined with respect to the insertion direction, and each line segment that connects the two holes 26 and 28 of the holder 1 is also inclined with respect to the insertion direction. Accordingly, when the microfluidic chips 2 are inserted into the slots 4, the communication holes 12, which are to be arranged in the deep sides of the slots 4, do not pass through an non-target hole, thereby reducing intrusion of air bubbles into the culture solution 50. The inclination angle of the line segment connecting the communication holes 10 and 12 with respect to the insertion direction is not limited, but is, for example, 15 degrees to 35 degrees.

Furthermore, on the lower surface of the upper wall 20 of the holder 1, a plurality of grooves 32 and 34 extending along the insertion direction of the slots 4 are formed. When the microfluidic chips 2 are inserted into the slots 4, the communication hole 10 of each microfluidic chip 2 moves in the slot 4 while overlapping with the groove 32, and finally reaches the target hole 26, whereas the communication hole 12 of each microfluidic chip 2 moves in the slot 4 while overlapping with the groove 34, and finally reaches the target hole 28 (see Fig. 19). When the microfluidic chip 2 is inserted into the slot 4, if the culture solution 50 filled in the communication holes 10 and 12 and the annular seals 36 and 38 and rising above the annular seals 36 and 38 touches the lower surface of the upper wall structure 19, the culture solution 50 moves in the slot 4 while continuously receiving a shearing force from the lower surface. Accordingly, part of the culture solution 50 continues to overflow from the annular seals 36 and 38, and when the communication holes 10 and 12 reach the target holes 26 and 28, the amount of the culture solution 50 in the communication holes 10 and 12 is significantly reduced, and air bubbles may intrude into the culture solution 50. In this embodiment, the communication holes 10 and 12 move in the slots 4 while overlapping the grooves 32 and 34 formed on the lower surface of the upper wall structure 19, and thus a time that the culture solution 50 is in contact with the lower surface of the upper wall structure 19 can be minimized, thereby preventing bubbles from intruding into the culture solution 50.

The width of the groove 32 is preferably greater than the diameter of the culture solution 50 stored in the annular seal 36. Furthermore, the width of the groove 32 is preferably greater than the outer diameter of the annular seal 36 so that the annular seal 36 is not struck by the lower surface of the upper wall structure 19 during movement of the microfluidic chip 2. The width of the groove 34 is preferably greater than the diameter of the culture solution 50 stored in the annular seal 38. Furthermore, the width of the groove 34 is preferably greater than the outer diameter of the annular seal 38 so that the annular seal 38 is not struck by the lower surface of the upper wall structure 19 during the movement of the microfluidic chip 2.

As shown in Fig. 1, the groove 32 is spaced apart from the hole 26, and the groove 34 is spaced apart from the hole 28. This is because the lower surface of the upper wall structure 19 applies a force to the annular seals 36 and 38 arranged around the holes 26 and 28 uniformly in the circumferential direction.

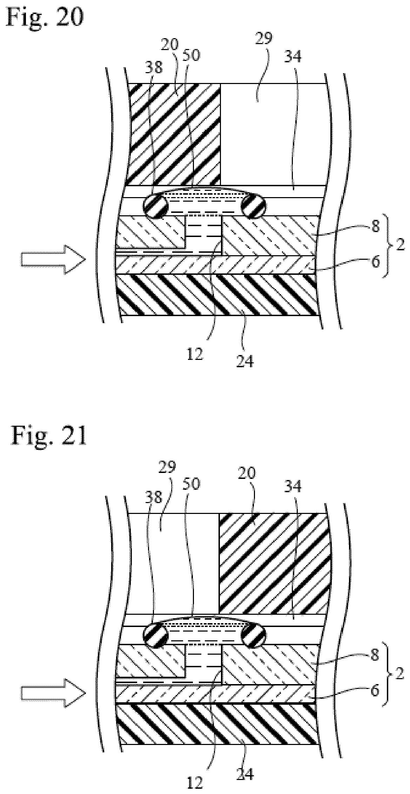

With reference to Figs. 19 to 23, the state of the culture solution 50 stored in the communication hole 12 to be arranged on the deep side of the slot 4 and in the annular seal 38 while the microfluidic chip 2 is inserted into the slot 4 will be described. As shown in Fig. 19, at the initial phase when the microfluidic chip 2 is inserted into the slot 4, the rising upper surface of the culture solution 50 does not come into contact with the lower surface of the upper wall 20 due to the existence of the groove 34. Next, as shown in Fig. 20, the communication hole 12 and the annular seal 38 reach the observation window 29. Since the observation window 29 penetrates the upper wall 20, when the communication hole 12 and the annular seal 38 pass through the observation window 29, the culture solution 50 filled in the communication hole 12 and the annular seal 38 becomes not to face the lower surface of the upper wall 20. Next, as shown in Fig. 21, the communication hole 12 and the annular seal 38 pass through the edge of the observation window 29 and face the lower surface of the upper wall 20 again, but since the groove 34 extends across the observation window 29, the rising upper surface of the culture solution 50 also does not touch the lower surface of the upper wall 20. Thus, at the phases shown in Figs. 19 to 21, the upper surface of the culture solution 50 continues to be spaced apart from the lower surface of the upper wall structure 19.

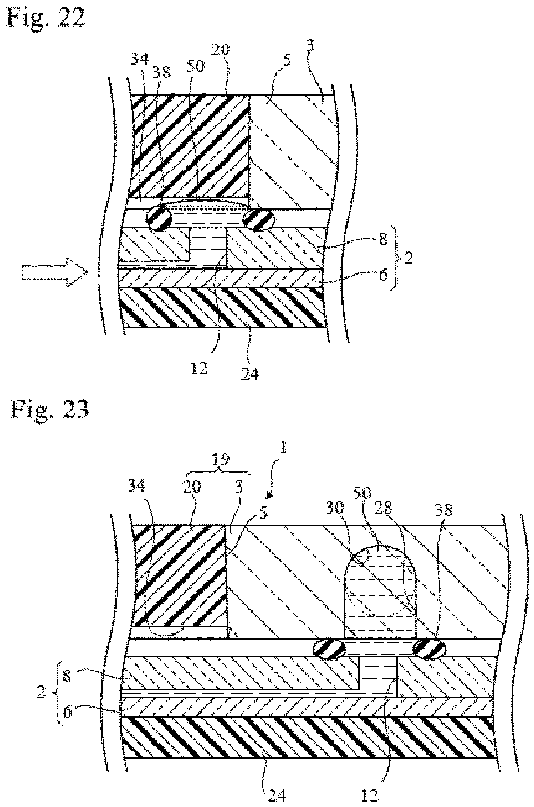

Next, as shown in Fig. 22, the communication hole 12 and the annular seal 38 reach the connection flow passage chip 3. At this time, the culture solution 50 stored in the communication hole 12 and the annular seal 38 is subjected to a shearing force by the lower edge of the connection flow passage chip 3, and a part of the culture solution 50 overflows from the annular seal 38. Immediately afterwards, the culture solution 50 is covered with the lower surface of the connection flow passage chip 3, and therefore, there is little possibility that air bubbles will intrude thereinto. Next, as shown in Fig. 23, the communication hole 12 and the annular seal 38 reach the target hole 28.

The upper wall structure 19 of the holder 1 includes the connection flow passage chip 3 to be fixed to the holder 1, and the connection flow passage 30 and the holes 28 are formed in the connection flow passage 30. As will be apparent from Fig. 23, it is possible to fix the connection flow passage chip 3 to the holder 1 after filling the connection flow passage 30 and the holes 28 with the culture solution 50 (for example, a culture solution containing neither cells nor drugs). Preferably, before the microfluidic chips 2 are inserted into the slots 4, the connection flow passage 30 of which the connection flow passage 30 and the holes 28 are filled with the culture solution 50 is fixed to the holder 1. Due to interfacial tension, the culture solution 50 is prevented from flowing down from the connection flow passage chip 3. Thereafter, while moving the microfluidic chips 2 horizontally, as shown in Fig. 23, the culture solution 50 filled in the communication holes 12 and the annular seals 38 is connected to the culture solution 50 filled in the connection flow passage 30 and the holes 28 of the connection flow passage chip 3. On the contrary, if the connection flow passage chip 3 is fixed to the holder 1 after the microfluidic chips 2 are inserted into the slots 4, when the connection flow passage chip 3 is moved downward, air bubbles may intrude into the culture solution 50.

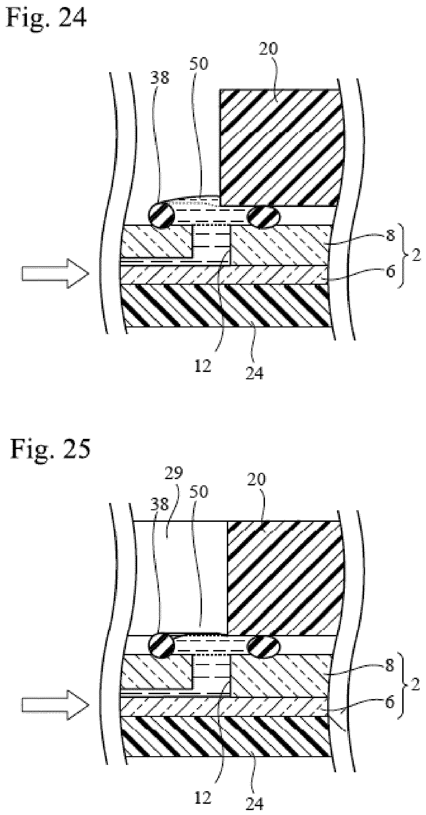

If the grooves 34 are not formed on the lower surface of the upper wall 20, as shown in Fig. 24 (corresponding to Fig. 19), at the initial phase when the microfluidic chips 2 are inserted into the slots 4, the rising upper surface of the culture solution 50 stored in the communication hole 12 and the annular seal 38 is subjected to a shearing force by the lower edge of the upper wall 20, and a part of the culture solution 50 overflows from the annular seal 38. Thereafter, the culture solution 50 moves in the slot 4 while continuously receiving the shearing force from the lower surface, so that part of the culture solution 50 continues to overflow from the annular seal 38. Furthermore, as shown in Fig. 25 (corresponding to Fig. 21), when the communication hole 12 and the annular seal 38 pass through the observation window 29, the culture solution 50 stored in the communication hole 12 and the annular seal 38 momentarily comes into contact with air at the observation window 29, and is subjected to a shearing force by the lower edge of the upper wall 20 again, so that a part of the culture solution 50 overflows from the annular seal 38. Thus, in a case in which the grooves 34 are not formed on the lower surface of the upper wall 20, the culture solution 50 is significantly reduced, and air bubbles may intrude into the culture solution 50.

On the other hand, in the embodiment, since the grooves 34 extend across the observation windows 29, when the culture solution 50 stored in the communication holes 12 and the annular seals 38 pass through the observation windows 29, it is possible to minimize the reduction of the amount of the culture solution 50 and intrusion of the air bubbles.

In the use of this organ-on-a-chip device, when the substance produced in the culture chamber 14 of one microfluidic chip 2 (for example, a microfluidic chip that simulates a liver) is transferred to another microfluidic chip 2 (for example, a microfluidic chip that simulates a lung), a new culture solution is injected into the through-hole 26 that communicates with the communication hole 10 of the former microfluidic chip 2. A syringe (e.g., a syringe pump) may be used to inject the new culture solution. By injecting the new culture solution into the through-hole 26 and the communication hole 10, the culture solution 50 containing the substance generated in the culture chamber 14 that simulates the liver is pushed by the new culture solution and flows into the communication hole 12 and the hole 28. The culture solution 50 containing the substance further passes through the connection flow passage 30, and flows into the culture chamber 14 that simulates the lung via the other hole 28 and the other communication hole 12. An excess amount of the culture solution 50 in the culture chamber 14 that simulates the lung is discharged from the communication hole 10 and the through-hole 26 that communicate with the culture chamber 14. The compressed annular seals 36 and 38 described above are capable of sealing the liquid. Furthermore, by transferring the culture solution 50 from the flow passage of one microfluidic chip 2 to the flow passage of another microfluidic chip 2 after filling the connection flow passage 30 and the holes 28 with the culture solution 50, it is possible to prevent air from entering the culture solution 50.

### Other Modifications

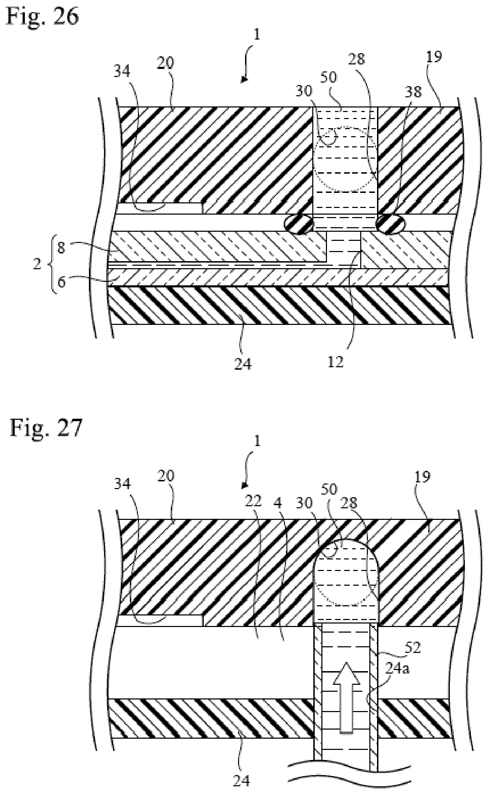

For example, in the embodiment, the upper wall structure 19 of the holder 1 includes, in addition to the upper wall 20, the connection flow passage chip 3 fixed to the holder 1, and the connection flow passage 30 and the holes 28 are formed in the connection flow passage 30. However, the connection flow passage chip 3 may be eliminated. In a modification shown in Fig. 26, the upper wall structure 19 has only the upper wall 20, in which the connection flow passage 30 and the holes 28 are formed. In this modification, the holes 28 are through-holes, and before the microfluidic chips 2 are inserted into the slots 4, the culture solution 50 is filled into the connection flow passage 30 and the holes 28 through a through-hole 28 from above the upper wall 20. Due to interfacial tension, the culture solution 50 is prevented from flowing down from the holes 28. Then, the culture solution 50 filled in the communication hole 12 and the annular seal 38 is connected to the culture solution 50 filled in the connection flow passage 30 and the holes 28 while moving the microfluidic chip 2 horizontally. When transferring the culture solution 50 from a microfluidic chip 2 to another microfluidic chip 2, upper parts of the holes 28 are blocked by plugs or blocking plates (not shown) so that the culture solution 50 does not flow out from the holes 28 opening upward.

In a modification shown in Fig. 27, the upper wall structure 19 has only the upper wall 20, in which the connection flow passage 30 and the holes 28 are formed. In this modification, the holes 28 open on the lower surface of the upper wall 20. A through-hole 24a is formed in the lower wall 24, and a syringe 52 is inserted into the through-hole 24a. Before the microfluidic chips 2 are inserted into the slots 4, the syringe 52 is used to fill the connection flow passage 30 and the holes 28 with the culture solution 50. Even if the pressure by the syringe 52 is released, due to interfacial tension, the culture solution 50 is prevented from flowing down from the hole 28. Thereafter, the syringe 52 is removed from the microfluidic device, and the culture solution 50 filled in the communication holes 12 and the annular seals 38 is connected with the culture solution 50 filled in the connection flow passage 30 and the holes 28 while moving the microfluidic chips 2 horizontally.

In the embodiment and the modifications, the connection flow passage 30 and the holes 28 are formed in the same member (the connection flow passage chip 3 or the upper wall 20). However, the connection flow passage 30 may be formed in a member different from the member in which the hole 28 is formed, and these members may be joined.

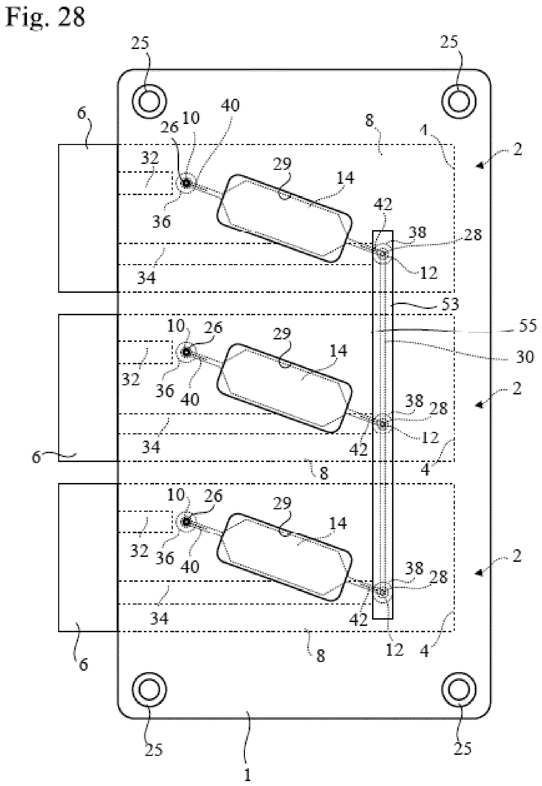

In the embodiment, two microfluidic chips 2 are inserted in the holder 1. However, three or more microfluidic chips 2 may be inserted in the holder 1. Fig. 28 shows a microfluidic device having three microfluidic chips 2. In this modification, an elongated opening 55 is formed in the upper part of the holder 1, and a connection flow passage chip 53 extending over three microfluidic chips 2 is fitted in the opening 55. The connection flow passage chip 53 has three holes 28 that communicate with the communication holes 12 of the three microfluidic chips 2, respectively, and a connection flow passage 30 that communicates with the three holes 28. Accordingly, in this modification, for example, the culture solution can be transferred from the central microfluidic chip 2 (simulating one organ) to the other two microfluidic chips 2 (simulating other two organs).

The microfluidic device according to the embodiment is an organ-on-a-chip device. However, the present invention may be applied to other microfluidic devices, such as other devices for analyzing animal body fluids or other liquids.

In the embodiment, a plurality of microfluidic chips 2 are horizontally inserted into a plurality of slots 4 of the holder 1 to form a microfluidic device. However, one microfluidic chip 2 may be inserted into one slot 4 of the holder 1 horizontally to form a microfluidic device.

In the embodiment, the grooves 32 and 34 are formed in the upper wall structure 19 of the holder 1. However, the grooves 32 and 34 are not absolutely necessary.

In the embodiment, annular seals are provided on the top of each microfluidic chip 2, and support structures are arranged in the flow passage. However, the support structures are not absolutely necessary. Furthermore, the annular seals are not absolutely necessary. In particular, as long as the grooves 32 and 34 are formed in the upper wall structure 19 of the holder 1, it is contemplated that the reduction amount of the liquid stored in the communication holes 10 and 12 is small when the microfluidic chips 2 are inserted into the slots 4 even if the annular seals are eliminated.

The above modifications may be combined as long as no contradiction arises thereby.

### REFERENCE SYMBOLS

1: Holder
2: Microfluidic chip
3: Connection flow passage chip
4: Slot
5: Opening
6: Flat plate
6a: Upper surface
6b: Lower surface
8: Flow passage plate
8a: Upper surface
8b: Lower surface
10, 12: Communication hole
14: Culture chamber
16, 18: Passage
19: Upper wall structure
20: Upper wall
22: Middle wall
24: Lower wall
26, 28: Hole
29: Observation window
30: Connection flow passage
32, 34: Groove
36, 38, 46, 48: Annular seal
40, 42: Support structure
50: Culture solution

## Claims

1. A microfluidic chip (2) for insertion into a slot (4) that extends horizontally, comprising:
an upper surface (8a);
a lower surface (6b) opposite the upper surface (8a);
a flow passage (14, 16, 18) for fluid located between the upper surface (8a) and the lower surface (6b); and
only two communication holes (10, 12) that communicate with the flow passage (14, 16, 18) and open at the upper surface (8a);
**characterized in that**
the flow passage (14, 16, 18) and the communication holes (10, 12) are adapted to store a liquid filled therein;
wherein a line segment that connects the two communication holes (10, 12) is inclined with respect to an insertion direction along which the microfluidic chip (2) is to be inserted into the slot (4).

2. The microfluidic chip (2) according to claim 1, wherein the two communication holes (10, 12) are arranged at respective ends of the flow passage (14, 16, 18), and wherein the flow passage (14, 16, 18) extends linearly along the line segment that connects the two communication holes (10, 12).

3. The microfluidic chip (2) according to claim 1 or 2, further comprising two annular seals (36, 38, 46, 48) formed from an elastomer, the two annular seals (36, 38, 46, 48) being fixed to the upper surface (8a) or being formed on the upper surface (8a) and surrounding the communication holes (10, 12), respectively, wherein the annular seals (36, 38, 46, 48) are adapted to store the liquid.

4. The microfluidic chip (2) according to claim 3, further comprising two support structures (40, 42) fixed at positions within the flow passage (14, 16, 18), the positions overlapping the annular seals (36, 38, 46, 48), the support structures (40, 42) securing the height of the flow passage (14, 16, 18) so that the flow passage (14, 16, 18) is not blocked.

5. A microfluidic device comprising:
at least one microfluidic chip (2) according to claim 1 or 2; and
a holder (1) in which the microfluidic chip (2) is held,
the holder (1) comprising:
at least one slot (4) into which the microfluidic chip (2) is inserted, the slot (4) extending horizontally;
an upper wall structure (19) facing the upper surface (8a) of the microfluidic chip (2) when the microfluidic chip (2) is inserted into the slot (4);
a lower wall (24) facing the lower surface (6b) of the microfluidic chip (2) when the microfluidic chip (2) is inserted into the slot (4); and
two holes (26, 28) formed at the upper wall structure (19), the two holes (26, 28) communicating with the two communication holes (10, 12) of the microfluidic chip (2), respectively, when the microfluidic chip (2) is inserted into the slot (4),
a line segment connecting the two holes (26, 28), which communicate with the two communication holes (10, 12) of the microfluidic chip (2), respectively, being inclined with respect to an insertion direction along which the microfluidic chip (2) is to be inserted into the slot (4).

6. A microfluidic device comprising:
at least one microfluidic chip (2) according to claim 3 or 4; and
a holder (1) in which the microfluidic chip (2) is held,
the holder (1) comprising:
at least one slot (4) into which the microfluidic chip (2) is inserted, the slot (4) extending horizontally;
an upper wall structure (19) facing the upper surface (8a) of the microfluidic chip (2) and compressing the annular seals (36, 38, 46, 48) toward the upper surface (8a) when the microfluidic chip (2) is inserted into the slot (4);
a lower wall (24) facing the lower surface (6b) of the microfluidic chip (2) when the microfluidic chip (2) is inserted into the slot (4); and
two holes (26, 28) formed at the upper wall structure (19), the two holes (26, 28) communicating with the two communication holes (10, 12) of the microfluidic chip (2), respectively, when the microfluidic chip (2) is inserted into the slot (4).

7. The microfluidic device according to claim 5 or 6, comprising at least two grooves (32, 34) formed on a lower surface (6b) of the upper wall structure (19) of the holder (1), the grooves (32, 34) extending linearly along an insertion direction along which the microfluidic chip (2) is to be inserted into the slot (4), the two holes (26, 28) existing on extension lines of the grooves (32, 34), respectively.

8. The microfluidic device according to claim 7, comprising at least one observation window (29) formed in and penetrating the upper wall structure (19) of the holder (1), the groove (34) corresponding to the extension line on which the hole (28) located on a deep side of the slot (4) exists extending across the observation window (29).

9. The microfluidic device according to any one of claims 5 to 8, comprising multiple microfluidic chips (2),
the holder (1) comprising:
multiple slots (4) into which the multiple microfluidic chips (2) are inserted, the slots (4) extending horizontally;
multiple holes (26, 28) communicating with multiple communication holes (10, 12) of the multiple microfluidic chips (2); and
a connection flow passage (30) formed in the upper wall structure (19), the connection flow passage (30) connecting the multiple holes (28) located on deep sides of the slots (4).

10. The microfluidic device according to claim 9, wherein the upper wall structure (19) comprises a connection flow passage chip (3) to be fixed to the holder (1), the connection flow passage (30) being formed on the connection flow passage chip (3).

## Patentansprüche

1. Mikrofluidchip (2) zum Einsetzen in einen sich horizontal erstreckenden Schlitz (4), der aufweist:
eine obere Fläche (8a);
eine untere Fläche (6b) gegenüber der oberen Fläche (8a);
einen Strömungskanal (14, 16, 18) für Fluid, der zwischen der oberen Fläche (8a) und der unteren Fläche (6b) angeordnet ist; und
nur zwei Verbindungslöcher (10, 12), die mit dem Strömungskanal (14, 16, 18) in Verbindung stehen und an der oberen Fläche (8a) münden;
**dadurch gekennzeichnet, dass**
der Strömungskanal (14, 16, 18) und die Verbindungslöcher (10, 12) geeignet sind, eine darin eingefüllte Flüssigkeit zu speichern;
wobei ein Liniensegment, das die beiden Verbindungslöcher (10, 12) verbindet, in Bezug auf eine Einführungsrichtung geneigt ist, entlang der der Mikrofluidchip (2) in den Schlitz (4) eingeführt werden soll.

2. Mikrofluidchip (2) nach Anspruch 1, wobei die beiden Verbindungslöcher (10, 12) an den jeweiligen Enden des Strömungskanals (14, 16, 18) angeordnet sind, und wobei sich der Strömungskanal (14, 16, 18) linear entlang des Liniensegments erstreckt, das die beiden Verbindungslöcher (10, 12) verbindet.

3. Mikrofluidchip (2) nach Anspruch 1 oder 2, der ferner zwei ringförmige Dichtungen (36, 38, 46, 48) aufweist, die aus einem Elastomer ausgebildet sind, wobei die beiden ringförmigen Dichtungen (36, 38, 46, 48) an der oberen Fläche (8a) befestigt sind oder auf der oberen Fläche (8a) ausgebildet sind und die Verbindungslöcher (10, 12) jeweils umgeben, wobei die ringförmigen Dichtungen (36, 38, 46, 48) zum Speichern der Flüssigkeit geeignet sind.

4. Mikrofluidchip (2) nach Anspruch 3, der ferner zwei Haltestrukturen (40, 42) aufweist, die an Positionen innerhalb des Strömungskanals (14, 16, 18) befestigt sind, wobei die Positionen die ringförmigen Dichtungen (36, 38, 46, 48) überlappen und die Haltestrukturen (40, 42) die Höhe des Strömungskanals (14, 16, 18) sichern, so dass der Strömungskanal (14, 16, 18) nicht blockiert wird.

5. Mikrofluidvorrichtung, die aufweist:
mindestens einen Mikrofluidchip (2) nach Anspruch 1 oder 2; und
einen Halter (1), in dem der Mikrofluidchip (2) gehalten wird,
wobei der Halter (1) aufweist:
mindestens einen Schlitz (4), in den der Mikrofluidchip (2) eingesetzt wird, wobei sich der Schlitz (4) horizontal erstreckt;
eine obere Wandstruktur (19), die der oberen Fläche (8a) des Mikrofluidchips (2) gegenüberliegt, wenn der Mikrofluidchip (2) in den Schlitz (4) eingeführt ist;
eine untere Wand (24), die der unteren Oberfläche (6b) des Mikrofluidchips (2) gegenüberliegt, wenn der Mikrofluidchip (2) in den Schlitz (4) eingesetzt ist; und
zwei Löcher (26, 28), die an der oberen Wandstruktur (19) ausgebildet sind, wobei die beiden Löcher (26, 28) jeweils mit den beiden Verbindungslöchern (10, 12) des Mikrofluidchips (2) in Verbindung stehen, wenn der Mikrofluidchip (2) in den Schlitz (4) eingesetzt ist,
ein Liniensegment, das die beiden Löcher (26, 28) verbindet, die jeweils mit den beiden Verbindungslöchern (10, 12) des Mikrofluidchips (2) in Verbindung stehen, das in Bezug auf eine Einführungsrichtung geneigt ist, entlang der der Mikrofluidchip (2) in den Schlitz (4) eingeführt werden soll.

6. Mikrofluidvorrichtung , die aufweist:
mindestens einen Mikrofluidchip (2) nach Anspruch 3 oder 4; und
einen Halter (1), in dem der Mikrofluidchip (2) gehalten wird,
wobei der Halter (1) aufweist:
mindestens einen Schlitz (4), in den der Mikrofluidchip (2) eingesetzt wird, wobei sich der Schlitz (4) horizontal erstreckt;
eine obere Wandstruktur (19), die der oberen Fläche (8a) des Mikrofluidchips (2) gegenüberliegt und die ringförmigen Dichtungen (36, 38, 46, 48) zur oberen Fläche (8a) zusammendrückt, wenn der Mikrofluidchip (2) in den Schlitz (4) eingeführt ist;
eine untere Wand (24), die der unteren Fläche (6b) des Mikrofluidchips (2) gegenüberliegt, wenn der Mikrofluidchip (2) in den Schlitz (4) eingesetzt ist; und
zwei Löcher (26, 28), die an der oberen Wandstruktur (19) ausgebildet sind, wobei die beiden Löcher (26, 28) jeweils mit den beiden Verbindungslöchern (10, 12) des Mikrofluidchips (2) in Verbindung stehen, wenn der Mikrofluidchip (2) in den Schlitz (4) eingesetzt ist.

7. Mikrofluidvorrichtung nach Anspruch 5 oder 6, die mindestens zwei Nuten (32, 34) aufweist, die auf einer unteren Fläche (6b) der oberen Wandstruktur (19) des Halters (1) ausgebildet sind, wobei sich die Nuten (32, 34) linear entlang einer Einführungsrichtung erstrecken, entlang der der Mikrofluidchip (2) in den Schlitz (4) eingeführt werden soll, und wobei die beiden Löcher (26, 28) jeweils auf Verlängerungslinien der Nuten (32, 34) vorhanden sind.

8. Mikrofluidvorrichtung nach Anspruch 7, die mindestens ein Beobachtungsfenster (29) aufweist, das in der oberen Wandstruktur (19) des Halters (1) ausgebildet ist und diese durchdringt, wobei die Nut (34), die der Verlängerungslinie entspricht, auf der das auf einer tiefen Seite des Schlitzes (4) befindliche Loch (28) vorhanden ist, sich über das Beobachtungsfenster (29) erstreckt.

9. Mikrofluidvorrichtung nach einem der Ansprüche 5 bis 8, die mehrere Mikrofluidchips (2) aufweist,
wobei der Halter (1) aufweist:
mehrere Schlitze (4), in die die mehreren Mikrofluidchips (2) eingesetzt werden, wobei sich die Schlitze (4) horizontal erstrecken;
mehrere Löcher (26, 28), die mit mehreren Verbindungslöchern (10, 12) der mehreren Mikrofluidchips (2) in Verbindung stehen; und
einen Verbindungsströmungskanal (30), der in der oberen Wandstruktur (19) ausgebildet ist, wobei der Verbindungsströmungskanal (30) die mehreren Löcher (28) verbindet, die sich auf den tiefen Seiten der Schlitze (4) befinden.

10. Mikrofluidvorrichtung nach Anspruch 9, wobei die obere Wandstruktur (19) einen Verbindungsströmungskanalchip (3) aufweist, der am Halter (1) befestigt werden soll, wobei der Verbindungsströmungskanal (30) auf dem Verbindungsströmungskanalchip (3) ausgebildet ist.

## Revendications

1. Puce microfluidique (2) destinée à être insérée dans une fente (4) à extension horizontale, comprenant :
une surface supérieure (8a) ;
une surface inférieure (6b) opposée à la surface supérieure (8a) ;
un passage d'écoulement (14, 16, 18) de fluide présenté entre la surface supérieure (8a) et la surface inférieure (6b) ; et
deux trous de communication (10, 12) seulement, communiquant avec le passage d'écoulement (14, 16, 18) et ouverts sur la surface supérieure (8a) ;
**caractérisée en ce que**
le passage d'écoulement (14, 16, 18) et les trous de communication (10, 12) sont adaptés au stockage d'un liquide versé à l'intérieur ;
un segment de ligne reliant les deux trous de communication (10, 12) étant incliné par rapport à une direction d'insertion dans laquelle la puce microfluidique (2) doit être insérée dans la fente (4).

2. Puce microfluidique (2) selon la revendication 1, où les deux trous de communication (10, 12) sont disposés aux extrémités respectives du passage d'écoulement (14, 16, 18), et où le passage d'écoulement (14, 16, 18) s'étend linéairement le long du segment de ligne reliant les deux trous de communication (10, 12).

3. Puce microfluidique (2) selon la revendication 1 ou la revendication 2, comprenant en outre deux joints annulaires (36, 38, 46, 48) constitués d'un élastomère, lesdits deux joints annulaires (36, 38, 46, 48) étant fixés à la surface supérieure (8a) ou étant formés sur la surface supérieure (8a) et entourant les trous de communication (10, 12) respectifs, lesdits joints annulaires (36, 38, 46, 48) étant adaptés pour le stockage du liquide.

4. Puce microfluidique (2) selon la revendication 3, comprenant en outre deux structures de support (40, 42) fixées à des emplacements à l'intérieur du passage d'écoulement (14, 16, 18), les emplacements chevauchant les joints annulaires (36, 38, 46, 48), les structures de support (40, 42) fixant la hauteur du passage d'écoulement (14, 16, 18) de manière à ne pas bloquer le passage d'écoulement (14, 16, 18).

5. Dispositif microfluidique, comprenant :
au moins une puce microfluidique (2) selon la revendication 1 ou la revendication 2 ; et
un support (1) où la puce microfluidique (2) est maintenue,
ledit support (1) présentant
au moins une fente (4) où la puce microfluidique (2) est insérée, ladite fente (4) s'étendant horizontalement ;
une structure de paroi supérieure (19) opposée à la surface supérieure (8a) de la puce microfluidique (2) lorsque la puce microfluidique (2) est insérée dans la fente (4) ;
une paroi inférieure (24) opposée à la surface inférieure (6b) de la puce microfluidique (2) lorsque la puce microfluidique (2) est insérée dans la fente (4) ; et
deux trous (26, 28) formés sur la structure de paroi supérieure (19), les deux trous (26, 28) communiquant avec les deux trous de communication (10, 12) respectifs de la puce microfluidique (2), lorsque la puce microfluidique (2) est insérée dans la fente (4),
un segment de ligne reliant les deux trous (26, 28) communiquant avec les deux trous de communication (10, 12) respectifs de la puce microfluidique (2),
et incliné par rapport à une direction d'insertion dans laquelle la puce microfluidique (2) doit être insérée dans la fente (4).

6. Dispositif microfluidique, comprenant :
au moins une puce microfluidique (2) selon la revendication 3 ou la revendication 4 ; et
un support (1) où la puce microfluidique (2) est maintenue,
ledit support (1) présentant
au moins une fente (4) où la puce microfluidique (2) est insérée, ladite fente (4) s'étendant horizontalement ;
une structure de paroi supérieure (19) opposée à la surface supérieure (8a) de la puce microfluidique (2) et comprimant les joints annulaires (36, 38, 46, 48) vers la surface supérieure (8a) lorsque la puce microfluidique (2) est insérée dans la fente (4) ;
une paroi inférieure (24) opposée à la surface inférieure (6b) de la puce microfluidique (2) lorsque la puce microfluidique (2) est insérée dans la fente (4) ; et
deux trous (26, 28) formés sur la structure de paroi supérieure (19), les deux trous (26, 28) communiquant avec les deux trous de communication (10, 12) respectifs de la puce microfluidique (2), lorsque la puce microfluidique (2) est insérée dans la fente (4).

7. Dispositif microfluidique selon la revendication 5 ou la revendication 6, comprenant au moins deux rainures (32, 34) formées sur une surface inférieure (6b) de la structure de paroi supérieure (19) du support (1), lesdites rainures (32, 34) s'étendant linéairement le long d'une direction d'insertion dans laquelle la puce microfluidique (2) doit être insérée dans la fente (4), les deux trous (26, 28) étant présentés sur des lignes d'extension respectives des rainures (32, 34).

8. Dispositif microfluidique selon la revendication 7, comprenant au moins une fenêtre d'observation (29) formée dans, et traversant la structure de paroi supérieure (19) du support (1), la rainure (34) correspondant à la ligne d'extension sur laquelle est présenté le trou (28) situé sur un côté profond de la fente (4), et s'étendant au travers de la fenêtre d'observation (29).

9. Dispositif microfluidique selon l'une des revendications 5 à 8, comprenant plusieurs puces microfluidiques (2),
le support (1) comprenant
plusieurs fentes (4) dans lesquelles sont insérées les plusieurs puces microfluidiques (2), lesdites fentes (4) s'étendant horizontalement ;
plusieurs trous (26, 28) communiquant avec plusieurs trous de communication (10, 12) des plusieurs puces microfluidiques (2) ; et
un passage d'écoulement de connexion (30) formé dans la structure de paroi supérieure (19), ledit passage de flux de connexion (30) reliant les multiples trous (28) situés sur des côtés profonds des fentes (4).

10. Dispositif microfluidique selon la revendication 9, où la structure de paroi supérieure (19) comprend une puce de passage d'écoulement de connexion (3) à fixer au support (1), le passage d'écoulement de connexion (30) étant formé sur la puce de passage d'écoulement de connexion (3).
